# EUROPEAN PATENT APPLICATION

(11) **EP 4 830 634 A1**
(43) Date of publication of application: **28.10.2026**
(21) Application number: 25172615.4
(22) Date of filing: 25.04.2025
(51) Int. Cl.: A61B 6/00, A61B 6/46

(54) **PROCESSING SPECTRAL COMPUTED TOMOGRAPHY DATA**

(71) Applicant: Koninklijke Philips N.V., 5656 AE Eindhoven (NL)
(72) Inventor: KLINDER, Tobias, Eindhoven (NL); NICKISCH, Hannes, Eindhoven (NL); BROSCH, Tom, Eindhoven (NL); WENZEL, Fabian, Eindhoven (NL)
(74) Representative: Philips Intellectual Property & Standards

(57) **Abstract**

A system (100) for processing spectral computed tomography, CT, data, is provided. The system includes one or more processors (110) configured to: generate, and output (S120) to a display device (130), a first image (140) representing an anatomical region within the subject. The first image is generated by processing a first energy selection from the spectral CT data (120) using a first algorithm. The processor(s) (110) are also configured to: generate, and output (S130) to the display device (130), one or more user-selectable sample images (150ᵢ). Each sample image represents at least a portion of the anatomical region and is generated by processing an energy selection from the spectral CT data (120) using a corresponding algorithm, and wherein the energy selection is different to the first energy selection and/or the algorithm is different to the first algorithm. The processor(s) (110) are also configured to: generate, and output (S140) to the display device (130), in response to a user selection of a sample image (150ᵢ), a further image (160) representing the anatomical region. The further image is generated by processing the energy selection corresponding to the selected sample image using the algorithm corresponding to the selected sample image.

## Description

### TECHNICAL FIELD

The present disclosure relates to processing spectral computed tomography, CT, data. A system, a computer-implemented method, and a computer program product, are disclosed.

### BACKGROUND

Spectral CT imaging is increasingly being used in clinical applications in which conventional CT imaging has traditionally been used. For instance, spectral CT imaging is currently used to evaluate aortic disease, to assess myocardial perfusion, and to detect pulmonary embolisms. The data that is used to generate spectral CT images defines X-ray attenuation within multiple different X-ray energy intervals. This "spectral" CT data may be processed, e.g. using various material decomposition algorithms, to provide a distinction between materials that have similar X-ray attenuation values when measured within a single energy interval, and which would be indistinguishable in "conventional" CT data. Consequently, spectral CT imaging offers great potential to improve confidence in clinical diagnoses, and to avoid overlooking, or mis-diagnosing, clinical conditions.

Despite the recognized benefits of spectral CT imaging, conventional CT imaging remains the default choice for many clinical applications. This is believed to be the result of factors that include the complexity, and also the amount, of information that can be obtained from spectral CT imaging; both of which can overwhelm clinicians. For instance, in order to perform a diagnosis of a suspected condition, a clinician may need to know firstly which type of spectral CT analysis to perform in order to visualize the clinical condition, and secondly which settings (e.g. the level, and the window) to use in order to visualize it in an optimal way. In many cases, the analysis of spectral CT data can therefore be overwhelming for clinicians. As a result, spectral CT data remains under-utilized, and many of its benefits are not exploited to their full potential.

Thus, there remains a need for improvements that support clinicians in the analysis of spectral CT data.

### SUMMARY

The invention is defined by the claims.

According to one aspect of the present disclosure, a system for processing spectral computed tomography, CT, data, is provided. The system includes one or more processors configured to:
receive spectral CT data representing X-ray attenuation within a subject;
generate, and output to a display device, a first image representing an anatomical region within the subject, the first image being generated by processing a first energy selection from the spectral CT data using a first algorithm;
generate, and output to the display device, one or more user-selectable sample images, each sample image representing at least a portion of the anatomical region and being generated by processing an energy selection from the spectral CT data using a corresponding algorithm, and wherein the energy selection is different to the first energy selection and/or the algorithm is different to the first algorithm; and
generate, and output to the display device, in response to a user selection of a sample image, a further image representing the anatomical region, the further image being generated by processing the energy selection corresponding to the selected sample image using the algorithm corresponding to the selected sample image.

The above-described system generates user-selectable sample image(s) that correspond to different types of analysis(es) of the spectral CT data. The type(s) of analysis(es) used to generate the user-selectable sample image(s) is different to the type of analysis used to generate the first image. In response to a user's selection of a sample image, a further image representing the anatomical region, is generated. The further image is generated by performing the same type of analysis on the spectral CT data as that used to generate the selected sample image. The sample image(s) therefore provide sample(s) of different types of analysis(es) that may be performed on the spectral CT data, and which when selected, result in further images being generated using a corresponding type of analysis. By providing such sample image(s), and by generating their corresponding further image(s), a user may readily view the results of a type of analysis that may be performed on the spectral CT data. This alleviates the burden on the user of having to determine for themself, which type of analysis to perform on the spectral CT data.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a system 100 for processing spectral computed tomography data 120, in accordance with some aspects of the present disclosure.
Fig. 2 is a flowchart illustrating an example of a computer-implemented method of processing spectral computed tomography data, in accordance with some aspects of the present disclosure.
Fig. 3 is an example of a display device 130 having displayed thereon a first image 140, and multiple sample images 150ᵢ, in accordance with some aspects of the present disclosure.
Fig. 4 is an example of a display device 130 having displayed thereon a first image 140, multiple sample images 150ᵢ, and a further image 160 that is generated in response to a user selection of the sample image 150₂, in accordance with some aspects of the present disclosure.
Fig. 5 is a first example illustrating multiple sample images 150ᵢ that are outputted based on the identification of a clinical finding of a suspected pathological condition within a region of interest 170₃, in accordance with some aspects of the present disclosure.
Fig. 6 is a second example illustrating multiple sample images 150ᵢ that are outputted based on the identification of a clinical finding of a suspected pathological condition within a region of interest 170₄, in accordance with some aspects of the present disclosure.

### DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer-implemented method, and in a computer program product, in a corresponding manner.

In the following description, reference is made to a method of processing spectral CT data. The spectral CT data represents X-ray attenuation within a subject. In some examples, reference is made to the spectral CT data representing X-ray attenuation in a section through the liver of the subject. However, it is to be appreciated that this serves only as an example, and that the spectral CT data may in general represent X-ray attenuation within any anatomical region in a subject.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed by the one or more processors of the system disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations that are performed by the one or more processors.

As mentioned above, there remains a need for improvements that support clinicians in the analysis of spectral CT data.

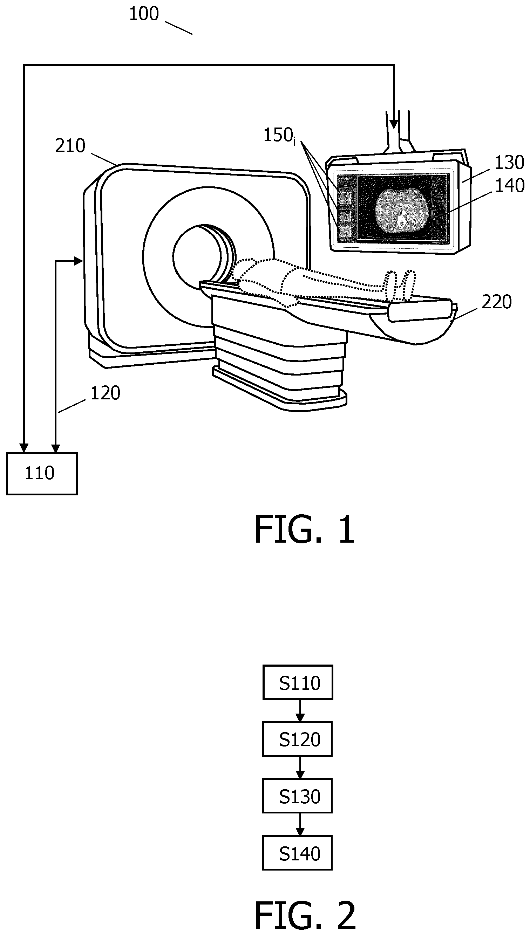

Fig. 1 is a schematic diagram illustrating an example of a system 100 for processing spectral computed tomography data 120, in accordance with some aspects of the present disclosure. Fig. 2 is a flowchart illustrating an example of a computer-implemented method of processing spectral computed tomography data, in accordance with some aspects of the present disclosure. It is noted that operations that are described as being performed by the one or more processors 110 of the system 100 illustrated in Fig. 1, may also be performed in the method illustrated in Fig. 2. Likewise, operations that are described in relation to the method described with reference to Fig. 2 may also be performed by the one or more processors 110 of the system 100 illustrated in Fig. 1. With reference to Fig. 1, and Fig. 2, the system 100 for processing spectral computed tomography, CT, data, includes one or more processors 110 configured to:
receive S110 spectral CT data 120 representing X-ray attenuation within a subject;
generate, and output S120 to a display device 130, a first image 140 representing an anatomical region within the subject, the first image being generated by processing a first energy selection from the spectral CT data 120 using a first algorithm;
generate, and output S130 to the display device 130, one or more user-selectable sample images 150ᵢ, each sample image representing at least a portion of the anatomical region and being generated by processing an energy selection from the spectral CT data 120 using a corresponding algorithm, and wherein the energy selection is different to the first energy selection and/or the algorithm is different to the first algorithm; and
generate, and output S140 to the display device 130, in response to a user selection of a sample image 150ᵢ, a further image 160 representing the anatomical region, the further image being generated by processing the energy selection corresponding to the selected sample image using the algorithm corresponding to the selected sample image.

The above-described system 100 generates user-selectable sample image(s) that correspond to different types of analysis(es) of the spectral CT data. The type(s) of analysis(es) used to generate the user-selectable sample image(s) is different to the type of analysis used to generate the first image. In response to a user's selection of a sample image, a further image representing the anatomical region, is generated. The further image is generated by performing the same type of analysis on the spectral CT data as that used to generate the selected sample image. The sample image(s) therefore provide sample(s) of different types of analysis(es) that may be performed on the spectral CT data, and which when selected, result in further images being generated using a corresponding type of analysis. By providing such sample image(s), and by generating their corresponding further image(s), a user may readily view the results of a type of analysis that may be performed on the spectral CT data. This alleviates the burden on the user of having to determine for themself, which type of analysis to perform on the spectral CT data.

The operations that are performed by the processor(s) 110 of the system 100 are described in more detail below.

Referring initially to the operation S110 illustrated in Fig. 2; in this operation, spectral CT data 120 is received. The spectral CT data 120 represents X-ray attenuation within a subject.

The spectral CT data 120 that is received in the operation S110 defines X-ray attenuation within the subject within each of a plurality of different energy intervals. In general there may be two or more energy intervals. By processing the data from the plurality of different energy intervals (e.g. using various material decomposition algorithms), a distinction can be made between materials that have similar X-ray attenuation values when measured within a single energy interval, and which would be indistinguishable in "conventional" CT data, i.e. data generated by a conventional CT imaging system, and which defines X-ray attenuation within a single energy interval.

The spectral CT data 120 that is received in the operation S110 may be generated by various configurations of spectral X-ray imaging systems, some of which are described below. In general, these include spectral CT imaging systems that acquire spectral X-ray attenuation data from each of a plurality of rotational angles with respect to an anatomical region whilst rotating an X-ray source-detector arrangement around the anatomical region, and also spectral X-ray projection imaging systems that are configured to acquire spectral X-ray attenuation data from each of a plurality of rotational angles with respect to an anatomical region. A spectral X-ray projection imaging systems is typically configured to acquire spectral X-ray attenuation data with its source-detector arrangement in a fixed position with respect to an anatomical region. However, the X-ray source-detector arrangement of a spectral X-ray projection imaging system may be configured to acquire spectral X-ray attenuation data from each of a plurality of rotational angles with respect to an anatomical region by stepping, or by rotating, its X-ray source-detector arrangement around the anatomical region.

In general, a spectral CT imaging system and a spectral X-ray projection imaging system are distinguished from their conventional CT imaging system and conventional X-ray projection imaging system counterparts by their ability to generate X-ray attenuation data within each of a plurality of different X-ray energy intervals. The set of spectral X-ray attenuation data that is acquired from a plurality of rotational angles by a spectral X-ray projection imaging system may be referred-to as spectral CT data because this dataset may be reconstructed to provide a volumetric image using similar techniques to those that are used to reconstruct the spectral CT data that is generated by a spectral CT imaging system.

In general, the X-ray source of the X-ray source-detector arrangement in the above-mentioned configurations of spectral X-ray imaging systems may be provided by multiple monochromatic sources, or by one or more polychromatic sources. In general, the X-ray detector in the X-ray source-detector arrangement in these configurations may include: a common detector for detecting X-ray radiation across multiple different X-ray energy intervals, or multiple detectors wherein each detector detects X-ray radiation within a different X-ray energy interval, or a multi-layer detector in which X-ray radiation within each of a plurality of different X-ray energy intervals is detected by corresponding layers (e.g. a so-called dual layer, or dual energy, detector), or a photon counting detector that bins detected X-ray photons into one of a plurality of different X-ray energy intervals based on their individual energies. In a photon counting detector, the relevant energy interval may be determined for each received X-ray photon by detecting the pulse height induced by electron-hole pairs that are generated in response to the X-ray photon's absorption in a direct-conversion material.

Various techniques may be used to generate X-ray attenuation data within each of a plurality of different X-ray energy intervals in the above-mentioned configurations. These include performing a temporal modulation of the X-ray spectrum; for example by temporally switching the X-ray anode potential of a single X-ray source, a technique known as "rapid kVp switching"; and temporally switching, or filtering, the emission of X-rays from multiple X-ray sources. Techniques that perform a temporal modulation of the X-ray spectrum may employ a common X-ray detector to detect X-rays across a plurality of different energy intervals, attenuation data for each of the energy intervals being generated in a time-sequential manner. Instead of performing a temporal modulation of the X-ray spectrum, a discrimination between different X-ray energy intervals may be provided at the X-ray detector by using a multi-layer detector, or a photon counting detector. Such detectors can detect X-rays from each of a plurality of different X-ray energy intervals near-simultaneously, and thus there is no need to perform temporal modulation of the X-ray spectrum. Thus, a multi-layer X-ray detector, or a photon counting X-ray detector, may be used in combination with a polychromatic X-ray source to generate spectral attenuation data within each of a plurality of different energy intervals.

Other configurations of the aforementioned X-ray sources and X-ray detectors may alternatively be used in a spectral CT imaging system, or in a spectral X-ray projection imaging system, to generate X-ray attenuation data within each of a plurality of different X-ray energy intervals. For example, in a yet further configuration, the need to sequentially switch different X-ray sources emitting X-rays in different energy intervals may be obviated by acquiring spectral X-ray attenuation data using two rotationally-offset X-ray source-detector arrangements. In this configuration, each X-ray source-detector arrangement acquires spectral X-ray attenuation data for a different X-ray energy interval, and so the spectral X-ray attenuation data for two different X-ray energy intervals may be acquired simultaneously.

In yet another configuration, spectral CT data 120 may be acquired from a conventional CT imaging system, or a conventional X-ray projection imaging system, by intercepting its X-ray beam with one or more spectral filters. For instance, a single filter may be mechanically moved into, and out of, the X-ray beam generated by a polychromatic X-ray source in order to temporally modulate the spectrum of X-rays that are detected by the corresponding X-ray detector. The filter transmits only a portion of the spectrum of X-rays emitted by the polychromatic X-ray source, and thus provides X-ray attenuation data for a first energy interval when the filter intercepts the beam. When the filter is removed from the beam, X-ray attenuation data is provided for a second energy interval, e.g. for the complete spectrum emitted by the polychromatic X-ray source. In so doing, X-ray attenuation data for each of the energy intervals is generated in a time-sequential manner. In this configuration, multiple filters, each with a different X-ray transmission spectrum, may be sequentially switched into, and out of, the X-ray beam in order to increase the number of energy intervals.

The spectral CT data 120 that is received in the operation S110 may represent X-ray attenuation within any anatomical region in the subject. By way of some examples, the spectral CT data 120 may represent X-ray attenuation within the subject's liver, or heart, or brain, or lung, or kidney, or arm, or leg, and so forth. In some examples, the spectral CT data represents X-ray attenuation within a portion of the subject's vasculature (e.g. within one or more blood vessels such as arteries, veins, arterioles, capillaries, venules, and so forth). The portion of the subject's vasculature may include a network of such blood vessels. In such examples, the spectral CT data 120 may be generated subsequent to the injection of a contrast agent into the vasculature. The contrast agent may include a substance such as Iodine, or a Lanthanide such as Gadolinium, or another substance that provides visibility of a blood flow into which the contrast agent is injected.

The spectral CT data 120 that is received in the operation S110 may be referred to as raw data, i.e. data that has not already been reconstructed into a volumetric image.

The spectral CT data 120 that is received in the operation S110 may be received from various sources. In some examples, the spectral CT data 120 is received from a computer readable storage medium. The computer readable storage medium may be in any location. For instance, it may be local to an imaging system that generates the spectral CT data, or it may be remote, e.g. in the Cloud. In one example, the spectral CT data 120 is received from a computer readable storage medium that serves as a Picture Archiving and Communication System, i.e. "PACS". In other examples, the spectral CT data 120 is received directly from an imaging system such as one of the imaging systems described above. The spectral CT data 120 may be received from the spectral CT imaging system 210 illustrated in Fig. 1, for example.

In general, the spectral CT data 120 that is received in the operation S 110 may be received via any form of data communication. For instance, the spectral CT data may be received via wired, or optical, or wireless communication. By way of some examples, when wired or optical communication is used, the communication may take place via signals transmitted on an electrical or optical cable, and when wireless communication is used, the communication may take place via RF or optical signals.

Referring now to the operation S120 illustrated in Fig. 2; in this operation, a first image 140 representing an anatomical region within the subject, is generated, and outputted S120 to a display device 130. The first image is generated by processing a first energy selection from the spectral CT data 120 using a first algorithm.

In general terms, the first image may be an objective image, or a material quantification image. Examples of objective images include mono-energetic images, or "MonoE" images, i.e. virtual monoenergetic images that approximate images that would result from the acquisition of X-ray photons with a specific single X-ray energy; Electron Density, or "%EDW" images that represent an estimate of effective atomic number based on spectral measurement; Z-Effective, or "EAN" images, i.e. images that represent an estimate of effective atomic number based on spectral measurement. Examples of material quantification images include Iodine images that represent the iodine concentration (e.g. in mg/ml); Uric Acid images that represent an estimate of the uric acid content; CaSupp images in which calcium content is suppressed via spectral analysis for better visualization of bone edema; virtual-non-contrast "VNC" images in which iodine content is suppressed; liver fat images that provide an estimate of liver fat via spectral analysis. In general, images such as these are generated by processing an energy selection from the spectral CT data using a corresponding algorithm The first image may be generated by using various known algorithms to process a corresponding energy selection. Some examples of such algorithms and their corresponding energy selections are described in a document by So, A., et al., "Spectral Computed Tomography: Fundamental Principles and Recent Developments" Korean J Radiol. 2021 Jan; 22(1):86-96.

In general, the first energy selection from the spectral CT data that is processed in order to generate the first image 140 in the operation S120 may be a subset of the spectral CT data 120, or it may be the complete set of spectral CT data 120. The subset may correspond to one or more predetermined X-ray energy intervals that are selected from the complete range of energy intervals. For example, in order to generate the above-described MonoE70 image, a "virtual" range of energy intervals centered on the X-ray energy of 70keV may be selected. Alternatively, the complete set of spectral CT data may be used to generate the first image. For instance, the first image may be a so-called conventional CT image that is generated by processing the X-ray attenuation data from all energy intervals.

The first image 140 may be outputted to various types of display devices 130 in the operation S120. For example, the first image 140 may be outputted to a monitor, or a display of a laptop, or a display of a tablet computer, or a display of a mobile communication device. By way of an example, the first image 140 may be outputted to the monitor of the monitor 130 illustrated in Fig. 1. The first image 140 may alternatively be outputted to other types of display devices, such as to a virtual/ augmented reality device. In some examples, the first image 140 represents an image slice(s) through the anatomical region. In other examples, the first image 140 represents the anatomical region in 3D, i.e. as a volumetric image.

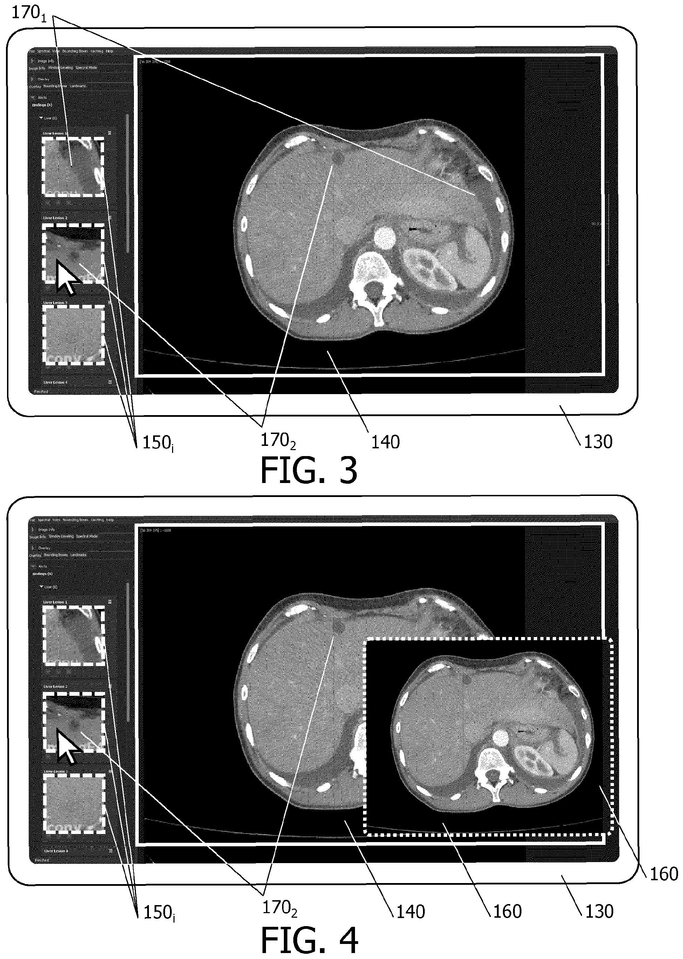

Fig. 3 is an example of a display device 130 having displayed thereon a first image 140, and multiple sample images 150ᵢ, in accordance with some aspects of the present disclosure. The first image 140 illustrated in Fig. 3 may be generated and displayed as a result of the operation S120 described above. In this example, the first image 140 is a conventional CT image that is generated from the complete set of spectral CT data. The first image 140 illustrated in Fig. 3 represents an image slice through a subject and includes portions of the subject's liver (located in the upper portion of the first image 140, the subject's ribs (surrounding the liver, and appearing as white dashes), and subject's spine (located in the lower central portion of the first image 140.

Referring now to the operation S130 illustrated in Fig. 2; in this operation, one or more user-selectable sample images 150ᵢ, is generated, and outputted to the display device 130. Each sample image 150ᵢ represents at least a portion of the anatomical region and is generated by processing an energy selection from the spectral CT data 120 using a corresponding algorithm. The energy selection is different to the first energy selection and/or the algorithm is different to the first algorithm.

The sample image(s) may be displayed on the display device in a variety of different ways in the operation S130. For instance, in some examples, the sample image(s) have a size (e.g. an area) that is less than a size of the first image 140. In these examples, the sample images may be displayed in the form of so-called thumbnail images. In some examples, the sample image(s) represent only a portion of the anatomical region that is represented in the first image 140, i.e. less than the amount of the anatomical region that is represented in the first image. In other examples, the sample image(s) represent the complete anatomical region that is represented in the first image 140. In these examples, image features may be represented in the sample image(s) with relatively lower resolution than the corresponding image features in the first image 140. In some examples, the sample image(s) represent volumetric renderings of the anatomical region. In some examples, the sample image(s) represent image slice(s) through the anatomical region. In some examples, the sample image(s) represent different perspectives of the anatomical region, i.e. views from different orientations. In some examples, the sample image(s) represent re-formatted versions of the anatomical region that is represented in the first image. For instance, elongated curved structures in the first image 140 may be re-formatted to provide using a curved planar reformation "CPR" to provide the sample image(s). An example of the CPR is described in the document by Kanitsar, A. et al., "CPR - Curved planar reformation". Proceedings of the IEEE Visualization Conference. 37-44. 10.1109/VISUAL.2002.1183754. The generation of sample image(s) that represent a combination of these examples is also contemplated.

As mentioned above, in some examples, multiple sample images are outputted in the operation S130. In these examples, some, or all of the sample images may represent the same part of the anatomy (e.g. the complete anatomical region that is represented in the first image 140, or a sub-portion thereof), or alternatively they may represent different parts of the anatomy (e.g. one sample image may represent a first region of interest in the lung, and another sample image may represent a second region of interest in the lung, or in another part of the anatomy).

In general, the sample image(s) may be generated using algorithms and corresponding energy selections such as those described above for generating the first image 140. Thus, the user-selectable sample image(s) 150ᵢ may include objective image(s) corresponding to one or more predetermined X-ray energy intervals and/ or material quantification image(s) corresponding to one or more predetermined material types. However, the energy selection that is processed in order to generate the sample image(s) is different to the first energy selection that is processed in order to generate the first image 140 and/or the algorithm that is used to generate the sample image(s) is different to the first algorithm that is used to generate the first image 140. Thus, the sample image(s) provide "samples" of different types of analysis(es) that may be performed on the spectral CT data. These sample types of analysis are different to the type of analysis used to generate the first image 140 in the operation S120.

The sample images 150ᵢ that are generated in the operation S130 are user-selectable, i.e. capable of being selected by a user. The sample images 150ᵢ may be selectable by a user via a variety of user-input devices, such as a keyboard, or a mouse, or a joystick, or a touchscreen, or a voice-controlled user-input device, and so forth.

Some examples of the above-described user-selectable sample images 150ᵢ, are illustrated on the left-hand side of Fig. 3. In Fig. 3, the three user-selectable sample images 150_{1..3} represent different portions of the liver. The sample images 150_{1..3} illustrated in Fig. 3 have been generated by processing subsets of the complete set of spectral CT data. These subsets differ from the complete set of spectral CT data that is processed in order to generate the first image 140 in the operation S120, and which in this example is a conventional CT image. Moreover, the sample images 150_{1..3} illustrated in Fig. 3 have been generated by processing their energy selections using corresponding algorithms, and these algorithms are different to the first algorithm that is used to generate the first image 140. The sample images 150_{1..3} in the example illustrated in Fig. 3 have been generated using material decomposition algorithms. By contrast, the first image 140, being a conventional CT image, is generated simply by mapping the (Hounsfield Units) X-ray attenuation values to greyscale values.

Referring now to the operation S 140 illustrated in Fig. 2; in this operation, a further image 160 representing the anatomical region is generated, and outputted, to the display device 130. The further image 160 is generated in response to a user selection of a sample image 150ᵢ.

The user selection of a sample image 150ᵢ may be received via the user-input device described above. For instance, with reference to Fig. 3, a user may use a mouse to position the cursor illustrated in Fig. 3 over the sample image 150₂, and click a selection button on the mouse to select this sample image.

With continued reference to the operation S140 illustrated in Fig. 2; the further image is generated by processing the energy selection corresponding to the selected sample image using the algorithm corresponding to the selected sample image. Thus, with continued reference to the example illustrated in Fig. 3, in the operation S140, the further image is generated using the same type of analysis that was used to generate the selected sample image 150₂.

An example of the generation of a further image is illustrated in Fig. 4, which is an example of a display device 130 having displayed thereon a first image 140, multiple sample images 150ᵢ, and a further image 160 that is generated in response to a user selection of the sample image 150₂, in accordance with some aspects of the present disclosure. The example illustrated in Fig. 4 corresponds to the example illustrated in Fig. 3, and additionally includes the further image 160. With reference to Fig. 3 and Fig. 4, in the operation S140, a user selects the sample image 150₂ (e.g. as described above), and in response to this selection, the further image 160 illustrated in Fig. 4 is generated.

In general, the further image that is generated in the operation S 140 includes at least the portion of the anatomical region that is represented in the selected sample image 150ᵢ. The further image 160 may show the at least a portion of the anatomical region that is represented in the selected sample image 150ᵢ in more detail than in the sample image. For instance, the further image 160 may show the at least a portion of the anatomical region that is represented in the selected sample image 150ᵢ, and a size of this portion may be greater than its size in the selected sample image 150ᵢ. This enables a user to view in greater detail the results of the type of analysis that was used to generate the selected sample image. Alternatively, the further image 160 may include more of the anatomical region than the portion of the anatomical region that is represented in the selected sample image 150ᵢ. For instance, the further image 160 may include the complete anatomical region that is represented in the first image 140. This enables a user to view the results of the selected type of analysis in the same anatomical context as the first image 140. It is also contemplated to show in the further image a proportion of the anatomical region that is between only the portion of the anatomical region that is represented in the selected sample image 150ᵢ, and the complete anatomical region that is represented in the first image 140. In some examples, the further image 160 has a size that is greater than or equal to a size of the first image 140. In other examples, the further image 160 has a size that is less than a size of the first image 140.

In the example illustrated in Fig. 4, the further image 160 that is generated in response to a user selection of the sample image 150₂, includes more of the anatomical region than the portion of the anatomical region that is represented in the selected sample image 150ᵢ. In this example, the further image 160 includes the complete anatomical region that is represented in the first image 140. This allows a user to view the results of the selected type of analysis in the same anatomical context as the first image 140.

In one example, at least one of the one or more user-selectable sample images 150ᵢ represents an image slice through the anatomical region. In this example, the further image 160 that is generated in response to a user selection of the at least one sample image, comprises an image slice through a same section of the anatomical region as the image slice represented in the sample image. In this example, the further image may be outputted such that it is aligned with the first image 140. This example helps a user to visualize the correspondence between the features represented in the image slices.

In a related example, the further image 160 that is generated in response to a user selection of the at least one sample image, is a user-selectable image slice through the same section of the anatomical region as the image slice represented in the sample image. Moreover, the user-selectable image slice is selectable by a user from a group of image slices comprising: the image slice through the same section of the anatomical region as the image slice represented in the sample image, and one or more adjacent image slices through adjacent sections of the anatomical region.

In this example, the system 100 enables a user to visualize the results of processing the spectral CT data using the same type of analysis, in multiple adjacent image slices. This enables a user to investigate the extent of a suspected pathological condition in a direction perpendicular to a plane of the image slices. The user may scroll through adjacent image slices, for example.

In some examples, the processor(s) output, to the display device 130, a user control in combination with the further image 160. The user control may be used to adjust the energy selection and/or a parameter of the algorithm that is used to generate the further image. The user control may be provided in the form of a so-called slider control, or a dial, or a dropdown list of selectable values, and so forth. The user control allows a user to adjust the energy selection, or the parameter, in order to investigate its effect on the further image 160. This may enable the user to elucidate pathological conditions that are hard to visualize. By way of some examples, the user control may be used to adjust the virtual kiloelectron volts (keV) value that is used to generate a virtual Monoenergetic image, or to adjust the amount of calcium suppression in a CaSupp image.

In some examples, the processor(s) output, to the display device 130, a scatter plot of underlying photo-scatter values in combination with the further image 160. Spectral information can be analyzed efficiently using such scatter plots. The photo-scatter values may be obtained from a so-called spectral base image "SBI" container, or alternatively from a low MonoE image (e.g. 50keV) and a high MonoE image (e.g. 200keV).

The further image 160 may be outputted to the display device 130 in various ways in the operation S130. For instance, in one example it may be outputted to a different portion of the display device 130 than the first image 140, e.g. to one side of the first image. In another example, the further image 160 may be at least partially overlaid onto the first image 140, as illustrated in the example in Fig. 4. The further image 160 may be completely overlaid onto the first image 140 such that it replaces the first image 140.

One or more additional operations may be performed by the one or more processors 110 of the system 100 described above with reference to Fig. 1 and Fig. 2, as described in the examples below.

In one example, the portion of the anatomical region that is represented in the sample image(s) 150ᵢ is determined based a region of interest that is identified by analyzing the spectral CT data 120. In this example, the processor(s) 110 are configured to:
analyze the spectral CT data 120 to identify a region of interest 170ₖ within the anatomical region; and
determine the least a portion of the anatomical region represented in at least one of the sample images 150ᵢ based on the identified region of interest.

In this example, the region of interest may correspond to a specific part of the anatomy and/or to a clinical finding of a suspected pathological condition. For instance, the region of interest may correspond to a specific part of the anatomy such as the liver, or the pancreas, or the kidney, and so forth; or to a specific blood vessel (e.g. the left coronary artery); or a specific bone (e.g. a vertebra). Such regions of interest may be identified by segmenting images that are reconstructed from the spectral CT data 120, and which include the anatomical region represented in the first image 140. Examples of known segmentation techniques that may be used to segment the images include thresholding, region growing, template matching, level sets, active contour modelling, neural networks, e.g. U-Nets, and so forth.

Alternatively, or additionally, in this example, the region of interest may correspond to a clinical finding of a suspected pathological condition. For instance, the region of interest may correspond to a clinical finding of a suspected pathological condition such as an (oncological) lesion, a musculoskeletal "MSK" fracture, a kidney stone, a brain bleed, a pulmonary embolism, a stenosis, and so forth. Such clinical findings may be identified by analyzing images that are reconstructed from the spectral CT data 120 (e.g. the first image 140), and which include the anatomical region represented in the first image 140. Examples of known image analysis techniques that may be used to analyze the images include machine vision-based techniques, and machine learning-based techniques. For instance, a neural network may be trained using multiple instances of images that include a specific clinical finding (e.g. a lesion, or an MSK fracture, and so forth), together with an expert-determined ground truth clinical finding, to identify the clinical finding in newly-presented images.

In these examples, the system 100 therefore guides a user to further investigate an automatically-determined region of interest.

In another example, instead of determining the portion of the anatomical region represented in the sample image(s) based a region of interest that is identified by analyzing the spectral CT data 120, the region of interest is identified by a user. In this example, the processor(s) 110 are configured to receive user input identifying a region of interest in the first image 140; and to determine the least a portion of the anatomical region represented in at least one of the sample images 150ᵢ based on the identified region of interest.

In this example, the system 100 facilitates a user to investigate a self-determined region of interest.

In this example, the user input may be received via a user-input device, such as one of the user-input devices described above. For instance, with reference to Fig. 3, a user may click a selection button on a mouse in order to identify as the region of interest a portion of the first image 140 surrounding the position of the mouse cursor. Following the user's selection, one or more sample images are generated, and outputted, and which include the region of interest. The user may alternatively use the mouse cursor to identify the region of interest in other ways, such as by using the mouse cursor to delineate a region of interest, or draw a predetermined shape (e.g. a rectangle) around a region of interest.

In the above examples, the least a portion of the anatomical region represented in at least one of the sample images 150ᵢ, is determined based on a region of interest. In these examples, the sample image(s) 150ᵢ may be defined so as to include at least the region of interest. The sample image(s) 150ᵢ may be defined so as to include the region of interest and also a specified margin around the region of interest, for example.

In a related example, the processor(s) 110 are configured to determine the energy selection and/or the corresponding algorithm used to generate the at least one of the sample images 150ᵢ, based on the (automatically-determined, or user-identified) region of interest 170ₖ.

In this example, if the region of interest includes a portion of the lung (or the liver), for instance, then a sample image may be generated using an energy selection and an algorithm that are suitable for generating a contrast agent image (e.g. a so-called iodine image). A sample image showing contrasted tissue in the lung (or the liver) may be useful in visualizing lung parenchyma (or liver tissue). By way of another example, if the region of interest includes bone, for instance, then a sample image may be generated using the three material decomposition algorithm described in a document by Schwaiger, B. J., "Three-material decomposition with dual-layer spectral CT compared to MRI for the detection of bone marrow edema in patients with acute vertebral fractures", Skeletal Radiol. 2018 May 25;47(11):1533-1540. This algorithm may be used to separate the measured attenuation coefficient at 50 keV, and at 200 keV, into the volume fractions of red marrow, yellow marrow, and hydroxyapatite, and to generate a sample image that distinguishes between these components, and which consequently assists a user in visualizing the bone. By way of another example, if the region of interest includes a portion of the liver, for instance, then a sample image may be generated using an energy selection and an algorithm that are suitable for generating a so-called liver fat map.

By using the region of interest to determine the energy selection, or to determine the corresponding algorithm that is used to generate the sample image(s), the system 100 provides sample(s) of different types of analysis that may be performed on the spectral CT data that are relevant to the region of interest. This alleviates the user from the burden of making these choices themself. A user may then select a sample type of analysis, via the sample image(s) 150ᵢ, whereupon the system 100 provides a more detailed analysis of the region of interest using the same type of analysis.

In this example, the energy selection and/or algorithm for processing the spectral CT data may be determined for a given region of interest by using the region of interest to consult a look-up table that provides for each of multiple regions of interest, an appropriate energy selection and/or algorithm. For instance, a type of the region of interest may be determined by segmenting the anatomical region and applying an anatomical atlas, and the type of the anatomical region used to consult the look-up table. Instead of using a look-up table, other techniques that provide an association between a given region of interest and an appropriate energy selection and/or algorithm for processing the spectral CT data, may alternatively be used. For example, a neural network may be trained to predict an appropriate energy selection and/or algorithm for processing the spectral CT data from an inputted region of interest. The neural network may be trained using training data that provides for each of multiple instances of regions of interest, a corresponding ground truth appropriate energy selection and/or algorithm.

In another related example, the processor(s) 110 are configured to determine the energy selection and/or the corresponding algorithm used to generate the at least one of the sample images 150ᵢ, based on a predetermined clinical finding of a suspected pathological condition within the region of interest 170ₖ.

In this example, if the clinical finding is an oncological lesion, for instance, then the following group of sample images may be generated using their corresponding energy selections and algorithms: a low MonoE image, a VNC image, and an IodineNoWater image. These images are useful in visualizing oncological lesions.

Similarly, if the clinical finding is a musculoskeletal fracture, for instance, then the following group of sample images may be generated using their corresponding energy selections and algorithms: a low MonoE image, a VNC image, and a Calcium suppression image. These images are useful in visualizing musculoskeletal fractures.

Similarly, if the clinical finding is a kidney stone, for instance, then the following group of sample images may be generated using their corresponding energy selections and algorithms: a low MonoE image, a VNC image, and a Uric acid image. These images are useful in visualizing kidney stones.

Similarly, if the clinical finding is a brain bleed, for instance, then the following group of sample images may be generated using their corresponding energy selections and algorithms: a low MonoE image, a VNC image, and an Edema image. These images are useful in visualizing brain bleeds.

Similarly, if the clinical finding is pulmonary embolism, for instance, then the following group of sample images may be generated using their corresponding energy selections and algorithms: a low MonoE image, and a Zeff image. These images are useful in visualizing pulmonary embolisms.

In these examples, the clinical findings may be identified using the techniques described above. The energy selections and corresponding algorithms for processing the spectral CT data for a given clinical finding may also be determined using the techniques described above. For example, the clinical finding may be used to consult a look-up table that provides for each of multiple clinical findings, an appropriate energy selection and/or algorithm.

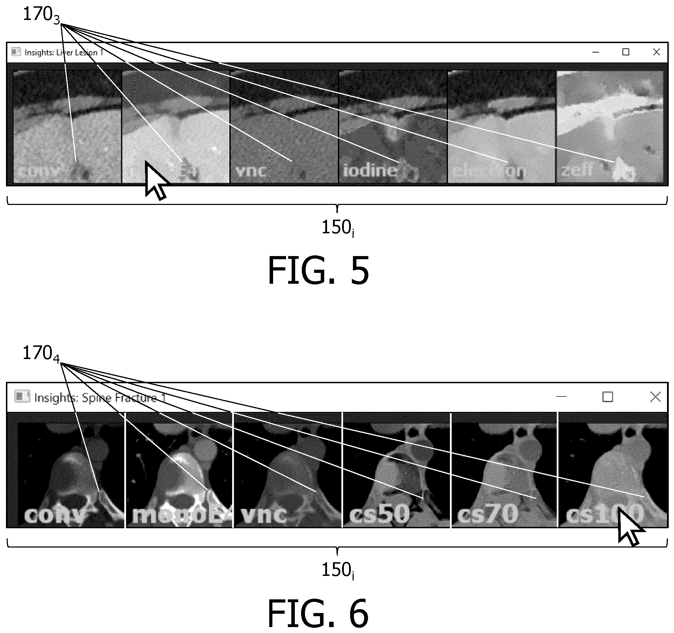

Some examples of sample images that are outputted based on the identification of a region of interest in the spectral CT data, are illustrated in Fig. 5, and Fig. 6. Fig. 5 is a first example illustrating multiple sample images 150ᵢ that are outputted based on the identification of a clinical finding of a suspected pathological condition within a region of interest 170₃, in accordance with some aspects of the present disclosure. In the example illustrated in Fig. 5, a clinical finding in the form of a liver lesion has been identified within the region of interest 170₃. The sample images 150ᵢ that are outputted in response to this finding correspond to (from left to right): a conventional CT image, a monoE40 image, a VNC image, an iodine image, an electron density image, and a Zeff image. The sample images illustrated in Fig. 5 are generated using energy selections and corresponding algorithms corresponding to these types of images.

Fig. 6 is a second example illustrating multiple sample images 150ᵢ that are outputted based on the identification of a clinical finding of a suspected pathological condition within a region of interest 170₄, in accordance with some aspects of the present disclosure. In the example illustrated in Fig. 6, a clinical finding in the form of a musculoskeletal spine fracture has been identified within the region of interest 170₄. The sample images 150ᵢ that are outputted in response to this finding correspond to (from left to right): a conventional CT image, a monoE40 image, a VNC image, a calcium suppression 50 image, a calcium suppression 70 image, and a calcium suppression 100 image. The value "X" in these calcium suppression "X" images refers to different levels of suppression, and originates in the slope of the calcium reference line in the photo-scatter domain that is used to generate the image. The sample images illustrated in Fig. 5 are generated using energy selections and corresponding algorithms corresponding to these types of images.

In one example, the processor(s) 110 are configured to automatically adjust an image contrast in the one or more user-selectable sample images 150ᵢ based on a determination of at least one of: a type of anatomy represented in the sample image, a view represented in the sample image, a presence of clinical finding of a suspected pathological condition in the sample image.

The dynamic range of image intensity values arising from different types of analysis has been found to vary depending on factors such as the type of anatomy represented in the sample image, a view represented in the sample image, and a presence of clinical finding of a suspected pathological condition in the sample image. Therefore, an improved visualization of the image contrast may be provided by automatically adjusting the image contrast in the sample images 150ᵢ based on these factors. For example, if the type of anatomy represented in the sample image is determined to be liver, then the image contrast (e.g. a level and/or window setting) may be set to predetermined values that are specific to liver. Similarly, if the view represented in a sample image is determined to be a sagittal view (e.g. for an MSK finding) then the image contrast may likewise be set to predetermined values that are specific to a sagittal view. Similarly, if it is determined that a lesion is present in the sample image, then the image contrast may likewise be set to predetermined values that are specific to the lesion.

In one example, the processor(s) 110 are configured to delineate the at least one region of interest 170ₖ in the first image 140 and/or in one or more of the user-selectable sample images 150ᵢ and/or in the further image 160.

Delineating the region of interest in accordance with this example helps to draw a user's attention to the region of interest, and consequently enables the user to review it efficiently. The region of interest may be delineated using image segmentation techniques such as those described above.

In one example, the processor(s) 110 generate, and to output, to the display device 130, a plurality of user-selectable sample images 150ᵢ that represent different regions of interest. In this example, the operation of outputting the user-selectable sample images to the display device 130 includes displaying the user-selectable sample images on the display device in groups according to a type of the region of interest 170ₖ.

This example also enables a user to review the regions of interest efficiently. The regions of interest may be grouped according to type of anatomy in the region of interest (e.g. liver, pancreas, kidney, bone, and so forth), or according to a type of clinical finding within the region of interest (e.g. lesion, MSK fracture, and so forth).

In one example, the operation of outputting the user-selectable sample images to the display device 130 includes identifying at least one sample image 150ᵢ as a preferred image for interpreting the spectral CT data 120. In this example, the processor(s) 110 are configured to determine the at least one sample image as a preferred image based on a comparison of one or more measurements in corresponding portions of the sample image and the first image 140.

In this example, the preferred image may be identified in various ways. For instance, the preferred image may be provided with a highlighted border, or text may be outputted indicating that this image is preferred. The measurements that are used to determine the preferred image may include image intensity measurements, or image contrast measurements, for example. For instance, a difference between the image contrast between a region corresponding to a lesion and a region corresponding to healthy tissue, may be compared for a sample image 150ᵢ and for the first image 140, in order evaluate the benefit in selecting the sample image to view the lesion instead of the first image. The sample image having the greatest benefit over the first image 140 may then be identified as the preferred image. This may be referred-to as identifying the preferred image based on the "spectral gain" of the sample images. In this example, a value indicative of the spectral gain may be outputted by the processor(s) in order to convey the benefit of performing this type of analysis on the spectral CT data. The value of the spectral gain may be calculated based on the difference between the image contrast between a region corresponding to a lesion and a region corresponding to healthy tissue for the preferred image. This quantification of the benefit of using a particular type of analysis may be used to convey to a user the benefit of performing this type of analysis on the spectral CT data instead of e.g. simply analyzing conventional CT data. An example situation in which the spectral gain may be used is in the assessment of iodine uptake in a lesion in order to quantify its malignancy. Iodine uptake may be quantitatively assessed by generating an Iodine image (i.e. no water) from spectral CT data. An average iodine uptake of less than 0.7 indicates that the lesion is benign, whereas a larger value is indicative an increased probability of the lesion being malignant. Segmentation can be used to determine iodine statistics within a defined boundary (e.g. within the lesion). Borderline cases that are around 0.7 are considered to benefit from their analysis being performed using spectral CT data.

In one example, the operation of outputting the one or more user-selectable sample images 150ᵢ comprises outputting an indication of an origin of the one or more user-selectable sample images in the first image 140.

By providing this indication, the system 100 enables a user to efficiently determine the correspondence between a sample image and the first image. The indication of the origin of the sample image(s) may be provided in various ways. For instance, colors, dashed lines, shading, and so forth, and which is common to both a sample image(s) and the first image 140 may be used to indicate the origin of a sample image. In a related example, the indication is provided in response to a user selection of a location within a sample image. For instance, if a user selects, using a user-input device, a location within a sample image, an icon may be temporarily shown in the corresponding location in the first image 140.

It is noted that in addition to the one or more processors 110, the system 100 may also include one or more of: a spectral X-ray imaging system, such as the spectral CT imaging system 210 illustrated in Fig. 1, for providing the spectral CT data 120; a display device 150, such as the monitor illustrated in Fig. 1, for displaying the outputs generated by the processor(s) 110; a patient bed 220; and a user-input device (not illustrated in Fig. 1) configured to receive user input in relation to the operations performed by the one or more processors 110, such as a keyboard, a mouse, a touchscreen, a voice-controlled user-input device, and so forth.

In another example, a computer-implemented method of processing spectral computed tomography, CT, data, is provided. The method includes:
receiving S110 spectral CT data 120 representing X-ray attenuation within a subject;
generating, and outputting S120 to a display device 130, a first image 140 representing an anatomical region within the subject, the first image being generated by processing a first energy selection from the spectral CT data 120 using a first algorithm;
generating, and outputting S130 to the display device 130, one or more user-selectable sample images 150ᵢ, each sample image representing at least a portion of the anatomical region and being generated by processing an energy selection from the spectral CT data 120 using a corresponding algorithm, and wherein the energy selection is different to the first energy selection and/or the algorithm is different to the first algorithm; and
generating, and outputting S140 to the display device 130, in response to a user selection of a sample image 150ᵢ, a further image 160 representing the anatomical region, the further image being generated by processing the energy selection corresponding to the selected sample image using the algorithm corresponding to the selected sample image.

In another example, a computer program product, is provided. The computer program product comprises instructions which when executed by one or more processors 110, cause the one or more processors to carry out a method of processing spectral computed tomography, CT, data. The method includes:
receiving S110 spectral CT data 120 representing X-ray attenuation within a subject;
generating, and outputting S120 to a display device 130, a first image 140 representing an anatomical region within the subject, the first image being generated by processing a first energy selection from the spectral CT data 120 using a first algorithm;
generating, and outputting S130 to the display device 130, one or more user-selectable sample images 150ᵢ, each sample image representing at least a portion of the anatomical region and being generated by processing an energy selection from the spectral CT data 120 using a corresponding algorithm, and wherein the energy selection is different to the first energy selection and/or the algorithm is different to the first algorithm; and
generating, and outputting S140 to the display device 130, in response to a user selection of a sample image 150ᵢ, a further image 160 representing the anatomical region, the further image being generated by processing the energy selection corresponding to the selected sample image using the algorithm corresponding to the selected sample image.

The above examples are to be understood as illustrative of the present disclosure, and not restrictive. Further examples are also contemplated. For instance, the examples described in relation to a system, may also be provided by the computer-implemented method, or by the computer program product, or by the computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for processing spectral computed tomography, CT, data, the system comprising one or more processors (110) configured to:
receive (S110) spectral CT data (120) representing X-ray attenuation within a subject;
generate, and output (S120) to a display device (130), a first image (140) representing an anatomical region within the subject, the first image being generated by processing a first energy selection from the spectral CT data (120) using a first algorithm;
generate, and output (S130) to the display device (130), one or more user-selectable sample images (150ᵢ), each sample image representing at least a portion of the anatomical region and being generated by processing an energy selection from the spectral CT data (120) using a corresponding algorithm, and wherein the energy selection is different to the first energy selection and/or the algorithm is different to the first algorithm; and
generate, and output (S140) to the display device (130), in response to a user selection of a sample image (150ᵢ), a further image (160) representing the anatomical region, the further image being generated by processing the energy selection corresponding to the selected sample image using the algorithm corresponding to the selected sample image.

2. The system according to any previous claim, wherein the one or more processors (110) are further configured to:
analyze the spectral CT data (120) to identify a region of interest (170ₖ) within the anatomical region; and
determine the least a portion of the anatomical region represented in at least one of the sample images (150ᵢ) based on the identified region of interest.

3. The system according to claim 2, wherein the one or more processors (110) are further configured to determine the energy selection and/or the corresponding algorithm used to generate the at least one of the sample images (150ᵢ), based on the identified region of interest (170ₖ).

4. The system according to any one of claims 2 - 3, wherein the one or more processors (110) are further configured to delineate the at least one region of interest (170ₖ) in the first image (140) and/or in one or more of the user-selectable sample images (150ᵢ) and/or in the further image (160).

5. The system according to any one of claims 2 - 4, wherein the one or more processors (110) are configured to generate, and to output, to the display device (130), a plurality of user-selectable sample images (150ᵢ), the sample images representing different regions of interest; and
wherein the outputting the user-selectable sample images to the display device (130) comprises displaying the user-selectable sample images on the display device in groups according to a type of the region of interest (170ₖ).

6. The system according to any previous claim, wherein the outputting the user-selectable sample images to the display device (130) comprises identifying at least one sample image (150ᵢ) as a preferred image for interpreting the spectral CT data (120); and
wherein the one or more processors (110) are configured to determine the at least one sample image as a preferred image based on a comparison of one or more measurements in corresponding portions of the sample image and the first image (140).

7. The system according to any previous claim, wherein the outputting the one or more user-selectable sample images (150ᵢ) comprises outputting an indication of an origin of the one or more user-selectable sample images in the first image (140).

8. The system according to any previous claim, wherein at least one of the one or more user-selectable sample images (150ᵢ) represents an image slice through the anatomical region, and wherein the further image (160) that is generated in response to a user selection of the at least one sample image, comprises an image slice through a same section of the anatomical region as the image slice represented in the sample image.

9. The system according to claim 8, wherein the further image (160) that is generated in response to a user selection of the at least one sample image, is a user-selectable image slice through the same section of the anatomical region as the image slice represented in the sample image, and wherein the user-selectable image slice is selectable by a user from a group of image slices comprising: the image slice through the same section of the anatomical region as the image slice represented in the sample image, and one or more adjacent image slices through adjacent sections of the anatomical region.

10. The system according to any previous claim, wherein the one or more user-selectable sample images (150ᵢ) comprises an objective image corresponding to one or more predetermined X-ray energy intervals, or wherein the one or more user-selectable sample images comprises a material quantification image corresponding to one or more predetermined material types.

11. The system according to any previous claim, wherein the first image (140) comprises a conventional CT image.

12. The system according to any previous claim, wherein the one or more processors (110) are further configured to automatically adjust an image contrast in the one or more user-selectable sample images (150ᵢ) based on a determination of at least one of: a type of anatomy represented in the sample image, a view represented in the sample image, a presence of clinical finding of a suspected pathological condition in the sample image.

13. The system according to any previous claim, wherein the one or more processors (110) are further configured to:
receive user input identifying a region of interest in the first image (140); and
determine the least a portion of the anatomical region represented in at least one of the sample images (150ᵢ) based on the identified region of interest.

14. A computer-implemented method of processing spectral computed tomography, CT, data, the method comprising:
receiving (S110) spectral CT data (120) representing X-ray attenuation within a subject;
generating, and outputting (S120) to a display device (130), a first image (140) representing an anatomical region within the subject, the first image being generated by processing a first energy selection from the spectral CT data (120) using a first algorithm;
generating, and outputting (S130) to the display device (130), one or more user-selectable sample images (150ᵢ), each sample image representing at least a portion of the anatomical region and being generated by processing an energy selection from the spectral CT data (120) using a corresponding algorithm, and wherein the energy selection is different to the first energy selection and/or the algorithm is different to the first algorithm; and
generating, and outputting (S140) to the display device (130), in response to a user selection of a sample image (150ᵢ), a further image (160) representing the anatomical region, the further image being generated by processing the energy selection corresponding to the selected sample image using the algorithm corresponding to the selected sample image.

15. A computer program product comprising instructions which when executed by one or more processors (110), cause the one or more processors to carry out the method according to claim 14.
